# EUROPEAN PATENT APPLICATION

(11) **EP 3 757 572 A1**
(43) Date of publication of application: **30.12.2020**
(21) Application number: 19305866.6
(22) Date of filing: 27.06.2019
(51) Int. Cl.: G01N 33/569, A61P 31/04, A61K 47/38, A61P 35/00

(54) **DETECTION OF PATHOGENIC BACTERIA BY CHEMICALLY FUNCTIONALIZED CELLULOSE**

(71) Applicant: Centre National de la Recherche Scientifique, 75016 Paris (FR); Université de Nantes, 44000 Nantes (FR); Universite Clermont Auvergne, 63000 Clermont Ferrand (FR)
(72) Inventor: GOUIN, Sébastien, 44470 THOUARE SUR LOIRE (FR); FELPIN, François-Xavier, 44800 ST HERBLAIN (FR); SIVIGNON, Adeline, 63360 GERZAT (FR); BARNICH, Nicolas, 63340 MADRIAT (FR)
(74) Representative: Novagraaf Technologies

(57) **Abstract**

The present invention concerns the rapid and selective detection of pathogenic bacteria expressing a lectin-type adhesin in a biological environment using a cellulose support functionalized with a ligand of a lectin-type adhesin, and its use for the diagnostic of infection with pathogenic bacteria expressing a lectin-type adhesin, for example in the context of Crohn's disease or in urinary tract infections.

## Description

### Technical field of the invention

The present invention concerns the rapid and selective detection in a biological environment of pathogenic bacteria expressing a lectin-type adhesin, in particular the phenotype of adherent and invasive *E. coli* (AIEC), using a cellulose support functionalized with a ligand of a lectin-type adhesin.

The present invention finds notably an application in the diagnosis of infections promoted by Fim-H-expressing pathogenic bacteria, for example in the context of urinary tract infections, osteoarticular infections and inflammatory bowel diseases such as Crohn's disease.

In the description below, the references in square brackets ([ ]) refer to the list of references at the end of the text.

### State of the art

Currently, the most effective way to treat a bacterial infection is to use antibiotics, which destroy bacterial community structure and can have also an impact on eukaryotic cell function. However, the accumulation of various factors such as excessive and non-targeted use of antibiotics and the lack of new compounds available is leading to the development of multidrug-resistant bacterial strains.

The worldwide dissemination of antibiotic resistances is a serious health problem, which requires a more rational use of antibiotics, the identification of new virulence factors and the development of alternative antibacterial therapeutics or diagnosis. Among the complementary anti-infective approaches, an anti-adhesive strategy consisting of preventing or blocking the adherence of bacteria to host cells through specific inhibitors, could be applied to address the infection in its first stage.

Indeed during an infectious process, pathogenic bacteria must first adhere to cell membranes before internalizing. Once the bacteria are installed in the cell, they can replicate and form intracellular colonies responsible for infectious recurrences and chronic infections. The adhesion step of bacteria to cells involves recognition between proteins (lectins) capable of specifically recognizing certain sugars present on the cell membrane. Thus an anti-adhesive strategy will allow to target bacteria with the targeted lectin, without interfering with other microorganisms. An anti-adhesive strategy will therefore requires the synthesis of lectin-specific inhibitors.

Pathogenic bacteria expressing a lectin-type adhesin, in particular certain *E.coli* strains expressing the FimH lectin at the top of their type 1 pili (or fimbriae), are involved in urinary tract infections (cystitis), osteoarticular infections (when placing prostheses), colorectal cancer and certain bowel diseases such as inflammatory bowel diseases (*i.e.* Crohn's disease). In this last pathology, adherent and invasive *E*. *coli* (AIEC for Adherent Invasive *Escherichia coli*) expressing the FimH lectin abnormally colonize the ileal mucosa of 25-60% of Crohn's disease patients and are within an ecological community of hundreds of symbiotic microorganisms but, when present, are suspected to play a major role in exacerbated inflammation in some patient cohorts (Carvalho et al., J. Exp. Med., 206: 2179-2189, 2009; Palmela et al., Gut, 67(3): 574-587, 2018) [1, 2].

The FimH adhesin expressed by pathogenic *E. coli* strains is able to recognize mannosides expressed on the surface of intestinal epithelial cells on CEACAM6 (CarcinoEmbryonic Antigen-related Cell Adhesion Molecule 6) glycoprotein, and is a particularly affine lectin for α-D-mannose. The FimH adhesin has been extensively studied as a target to disrupt the bacterial attachment to the host cells (Hartmann and Lindhorst, Eur. J. Org. Chem., 2011(20-21): 3583-3609, 2011) [3]. Results were obtained in the context of urinary tract infections (UTI), a prevalent infection generally mediated by the attachment of uropathogenic *E. coli* strains (UPEC) to highly mannosylated uroplakin cells. Orally administered FimH antagonists in *in vivo* UTI mouse models, were shown to decrease the *E. coli* load in the bladder by several orders of magnitude (Klein et al., J. Med. Chem., 53: 8627-8641, 2010; Kleeb et al., J. Med. Chem., 58: 2221-2239, 2015; Mydock-McGrane et al., J. Med. Chem., 59: 9390-9408, 2016) [4-6], competing with conventional antibiotic treatment (Jiang et al., J. Med. Chem., 55: 4700-4713, 2012) [7].

It has been recently shown that compounds (synthetic derivatives of heptylmannoside (HMan), a nanomolar FimH antagonist; Bouckaert et al., Mol. Microbiol., 55: 441-455, 2004 [8]) capable of blocking and removing AIECs from intestinal cells, could reduce signs of colitis and gut inflammation when administered *per* os (10 mg/kg) in mouse models mimicking Crohn's disease (Dorta et al., ChemBioChem., 17: 936-952, 2016; Sivignon et al., mBio, 2015, 6, e01298-15) [9, 10]. In this type of strategy, for proper stratification of patients harbouring *E. coli* pathovars before treatment, it would be essential to identify in advance the patients with positive AIEC to personalize such anti-adhesive treatment.

Currently, the detection of *E. coli* can be performed by global analysis of the intestinal bacterial metagenome. However, not all strains of *E. coli* are classified as pathogenic bacteria, and a large number of healthy subjects are carriers of non-pathogenic commensal *E. coli* strains. Furthermore no specific biomarkers are currently effective to distinguish certain pathogenic *E. coli,* such as AIEC from commensal *E. coli* in the complex gut microbiota. Their selective detection is thus carried out by phenotypic analyses of *E. coli* isolated on selective media on intestinal cell lines *in vitro,* but this is a time-consuming method which is difficult to apply in clinical practice.

There is therefore a real need for a new method allowing a rapid, non-invasive and selective detection of lectin-type adhesin expressing pathogenic bacteria, for example *E. coli* bacteria, and in particular from the adherent phenotype of AIECs, in a biological environment, and overcoming the defects, disadvantages and obstacles of the previous techniques.

### Description of the invention

Therefore the Inventors had the idea of trapping pathogenic bacteria expressing a lectin-type adhesin to a cellulosic framework by grafting ligand of lectin-type adhesin onto cellulose paper. It is then sufficient to use a revelation system (*e.g*. western-blot type using a chromogenic substrate, grinding the paper and extracting the trapped bacteria before spreading in a culture medium, metabolic detection of an enzyme specifically expressed by the trapped bacteria, etc...) to show that bacteria have adhered to the paper.

Cellulose fibers are cheap and biocompatible materials composed of β1-4 linked D-glucose units, provide a heterogeneous support unable to pass through the intestinal barriers thus avoiding possible side effects during interaction with other mannose-binding proteins, and are stable against enzymatic hydrolysis insofar as humans do not possess any enzyme capable of breaking the β-1,4-linked glucosyl units.

To this aim, the Inventors have developed modified cellulose papers or nanofibers (nanocellulose) so that they can specifically trap and accumulate pathogenic bacteria expressing a lectin-type adhesin, in particular AIEC pathogenic bacteria, and therefore allow their specific detection, even in biological fluids containing many bacterial species (*e.g*. urine, homogenised feces and intestinal tissues).

The bacterial adhesion of AIEC strains in the intestine being mainly promoted by a lectin-type adhesin recognizing mannose (FimH), the Inventors have grafted synthetic mannosides (HMan) with high affinity for FimH on cellulose papers. The sugar units that make up cellulose have been chemically activated to allow the grafting of functionalized ligands.

The first *in vitro* tests performed by the Inventors showed that the adhesion of AIECs to modified cellulose (MC) was dependent on the nature of the ligand grafted. The ligands with the highest affinity make it possible, once grafted onto cellulose, to capture more effectively the AIEC. MC has also been shown to be superior to bare cellulose (C) in the retention of AIECs. On the other hand, the Inventors showed that AIEC mutant bacteria deleted from the *fimH*gene as well as non-pathogenic and low adhesive *E. coli* bacteria (strain K12 C600) were very poorly retained on cellulose.

An analysis on mouse feces previously infected with AIEC bacteria showed that MC did not retain enterobacteria from the mouse microbiota but has been effective in trapping AIEC bacteria. Finally, AIEC bacteria associated to the colon of mice were preferentially retained on MC compared to the binding ability of the C.

These results are therefore particularly promising in a diagnostic application, for example in the context of Crohn's disease or in the urinary tract infections context. In this last case, the detection would be done by the general practitioner who could then (without antibiotic susceptibility testing) directly decide to the prescription of antibiotics. The strips of functionalized cellulose dipped in urine would concentrate the *E. coli.* Detection would then be by colorimetry after enzymatic hydrolysis of a chromogenic substrate whose product would be colored (J. Vis. Exp. (88), e5141414, doi:10.3791/5141414 (2014)) [11]. Paper functionalization with other types of sugars may be extended to other life-threatening pathogen expressing specific adhesins such as *Candida albicans,* Influenza, *Candida glabrata, Burkholderia species, Pseudomonas aeruginosa* or *Vibrio cholerae.*

An object of the present invention is therefore a method for detecting *in vitro* pathogenic bacteria expressing a lectin-type adhesin in a subject biological sample, comprising:
a) grafting at least one ligand of a lectin-type adhesin on a cellulose support;
b) contacting the functionalized-cellulose support with the subject biological sample under condition allowing selective adhesion of the pathogenic bacteria expressing the lectin-type adhesin thereon ;
c) detecting the pathogenic bacteria expressing the lectin-type adhesin trapped on the functionalized-cellulose support.

According to a particular embodiment of the method for detecting of the present invention, the lectin-type adhesin is FimH adhesin.

According to a particular embodiment of the method for detecting of the present invention, the ligand of the lectin-type adhesin is a mannose derivative, preferably a heptylmannoside (HMan), thiazolylmannoside,phenylmannoside, biphenylmannoside, etc..., or a derivative thereof.

According to a particular embodiment of the method for detecting of the present invention, the subject biological sample to be tested is chosen from the group consisting of bacterial culture, feces, urine, intestinal tissues.

According to a particular embodiment of the method for detecting of the present invention, the pathogenic bacteria expressing the lectin-type adhesin are Adherent Invasive *E. coli* (AIECs).

Another object of the present invention is a method for identifying a host having a pathology caused by pathogenic bacteria expressing a lectin-type adhesin and mediated by interactions between lectin-type adhesin and host cell surface glycans, comprising using the method for detecting of any one of claims 1 to 5.

According to a particular embodiment of the method for identifying of the present invention, the pathology is chosen in the group consisting of:
- an inflammatory bowel disease (such as Crohn's disease, ulcerative colitis, acute diarrhea);
- an urinary tract infection;
- an irritable bowel syndrome;
- colorectal cancer;
- infectious diarrhea.

According to a particular embodiment of the method for identifying of the present invention, the lectin-type adhesin is FimH adhesin.

According to a particular embodiment of the method for identifying of the present invention, the host is a human, a domestic mammal (such as dog or cat), or cattle such as pig, poultries (or swine).

Another object of the present invention is also a kit for detecting pathogenic bacteria expressing a lectin-type adhesin in a subject biological sample, comprising at least a cellulose support functionalized with a ligand to lectin-type adhesin, and at least a mean for detecting trapped pathogenic bacteria expressing the lectin-type adhesin. For example, a mean for detecting trapped pathogenic bacteria expressing the lectin-type adhesin is chosen from colorimetric, fluorescence, luminescence, and radioactivity detection. Preferably, a mean for detecting trapped pathogenic bacteria expressing the lectin-type adhesin is colorimetric or fluorescence or luminescence detection.

According to a particular embodiment of the kit of the present invention, the pathogenic bacteria expressing a lectin-type adhesin are Adherent Invasive *E. coli* (AIECs).

According to a particular embodiment of the method for identifying of the present invention, the lectin-type adhesin is FimH adhesin.

Another object of the present invention is also a cellulose support functionalized with a ligand of lectin-type adhesin for use in the treatment or prevention of a pathology caused by pathogenic bacteria expressing a lectin-type adhesin and mediated by interactions between lectin-type adhesin and host cell surface glycans.

According to a particular use of the functionalized-cellulose support of the present invention, the pathology is chosen in the group consisting of:
- an inflammatory bowel disease (Crohn's disease, ulcerative colitis, acute diarrhea);
- an urinary tract infection;
- an irritable bowel syndrome;
- colorectal cancer;
- infectious diarrhea.

According to a particular use of the functionalized-cellulose support of the present invention, the lectin-type adhesin is FimH adhesin.

### Brief description of the figures

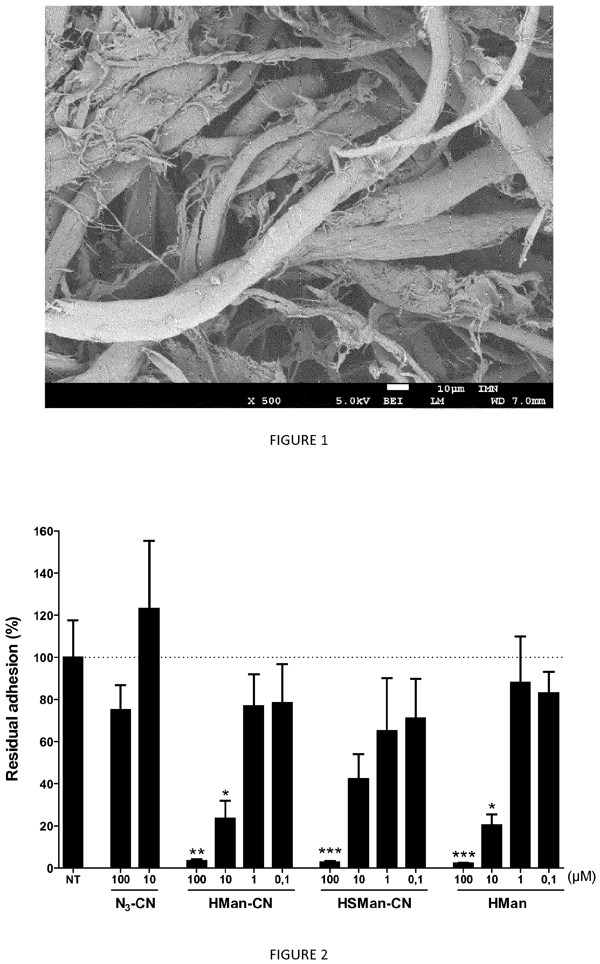

- Figure 1 represents a SEM analysis of HMan-CP showing that the structural integrity of the cellulose fibers is conserved after the chemical grafting.
- Figure 2 represents the effects of N₃-CN, HMan-CN, HSMan-CN and HMan on the adhesion ability of AIEC bacteria to T84 intestinal epithelial cells. Results are expressed as percentages of bacteria associated with the cells (n=6 experiments, means±SEMs; *p<0.05, **p<0.01, ***p<0.001). LF82 infection in the absence of treatment was normalized to 100 %. Treatments are tested at different concentrations, in micromolar (µM) on a mannose basis unit.

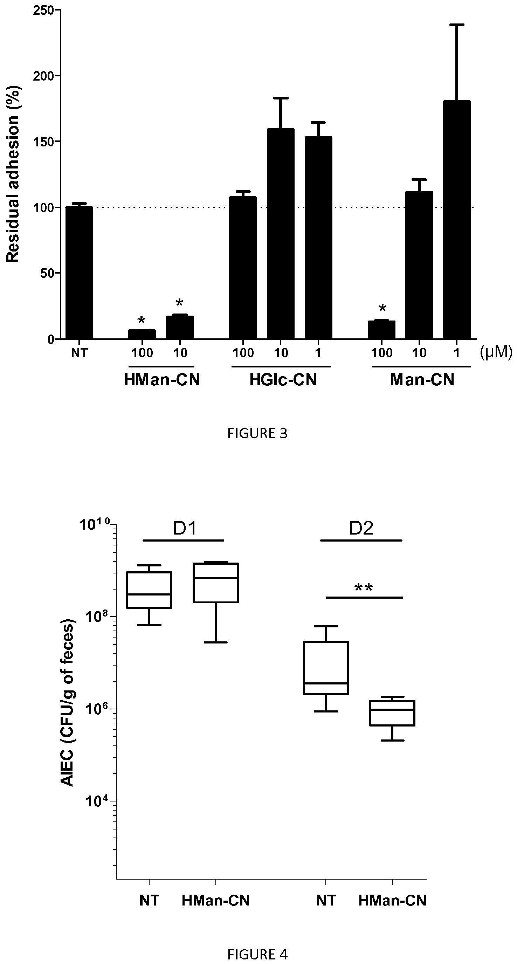

- Figure 3 represents the effects of HMan-CN, HGlc-CN and Man-CN on the adhesion ability of AIEC bacteria to T84 intestinal epithelial cells. Results are expressed as percentages of bacteria associated with the cells (n=6 experiments, means±SEMs; *p<0.05). LF82 infection in the absence of treatment (NT) was normalized to 100 %. Treatments are tested at two or three different concentrations, in micromolar (µM) on a mannose basis unit.
- Figure 4 represents the decreases of AIEC bacterial loads (logarithmic scale) in feces on day 1 (D1) and day 2 (D2) post-infection of CEABAC10 transgenic mice. HMan-CN was orally administered 2 h and 24 hours after the bacterial challenge of mice (10 mg/kg, on a mannose basis unit).

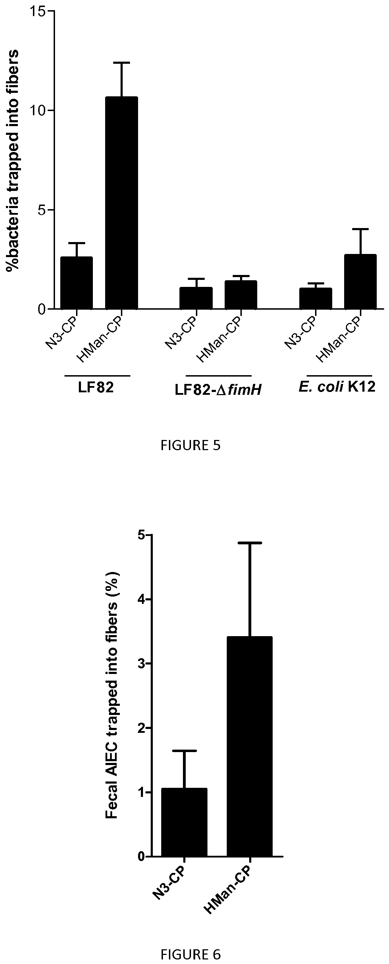

- Figure 5 represents the percentages of bacteria trapped by N₃-CP or HMan-CP. from bacterial suspensions prepared at 10⁸ bacteria/mL from an overnight culture in Luria Bertani broth medium. AIEC LF82-ΔfimH is a non-piliated mutant, unable to bind to intestinal epithelial cells.
- Figure 6 represents the percentages of fecal AIEC LF82 bacteria trapped by N₃-CP or HMan-CP. Fecal samples from LF82-infected CEABAC10 mice were homogenised, centrifuged and the supernatants were incubated with N₃-CP or HMan-CP. The percentages of trapped bacteria are defined according to the total number of AIEC bacteria presents in the biological samples.

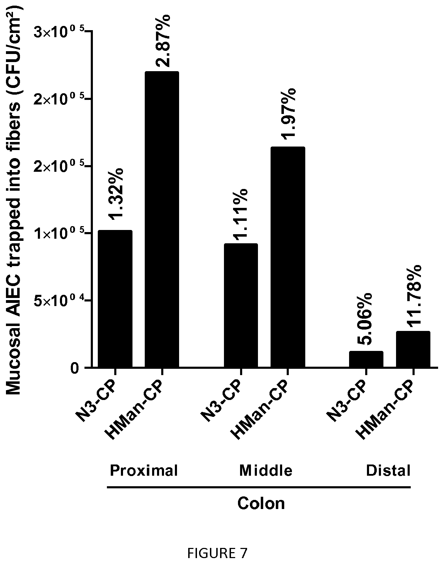

- Figure 7 represents the number of AIEC LF82 bacteria from intestinal tissues (in colony forming unit/cm² of cellulose) trapped by N₃-CP or HMan-CP. Intestinal tissues were homogenised, centrifuged and the supernatants were incubated with N₃-CP or HMan-CP. AIEC bacteria bound to the cellulose were quantified on antibiotic selective cultured media. The percentages of trapped bacteria are defined according to the total number of AIEC bacteria presents in the biological samples (indicated on the top of the bars).

### EXAMPLES

### EXAMPLE 1 : MANNOSE-FUNCTIONALIZED CELLULOSE FOR THE BINDING AND SPECIFIC DETECTION OF AIEC

**N₃-CP** fibers (disc of 5 mm diameter) were prepared as well as **HMan-CP** (disc of 5 mm diameter) by chemical functionalization of cellulose paper following four synthesis steps.

### General Methods

All reagents were purchased from Acros Organics or Aldrich and were used without further purification. Whatman grade 6 filter paper (42.5 mm Ø) with a grammage of ca. 100 g/m² was used as cellulose source. All compounds were fully characterized by ¹H (400.133 or 300.135 MHz), ¹³C (125.773 or 75.480 MHz) NMR spectroscopy (Bruker Avance 300 Ultra Shield or Bruker Avance III 400 spectrometer). When needed, 13 C heteronuclear HMQC and HMBC were used to unambiguously establish structures. High-resolution mass spectra (HRMS) were recorded with a Thermofisher hybrid LTQ-orbitrap spectrometer (ESI +) and a Bruker Autoflex III SmartBeam spectrometer (MALDI). FT-IR spectra were recorded on a Bruker Tensor 27 spectrometer with ATR technic and KBr tablet method. Elemental analyses were performed on a Thermo Fisher Scientific Flash 2000 CHNS organic elemental analyzer. Centrifugations were performed on a Sigma 3-16 Centrifuge. Dialysis were performed with Spectra/Por dialysis membrane MWCO 3500 K. Scanning electron microscopy (SEM) images were recorded with a JEOL 7600 F.

### Chemical synthesis of compounds 3 and 4

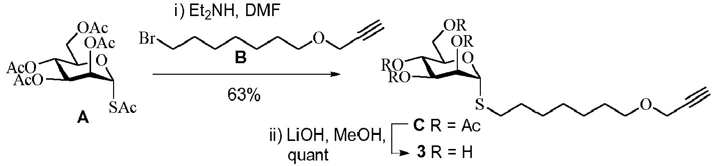

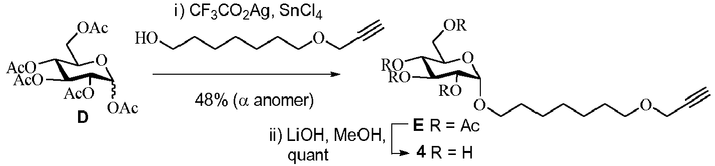

### 7 -Propargyloxyheptyl 1-thio-2,3,4,6-tetra-O-acetyl-α-D-mannopyranoside (C)

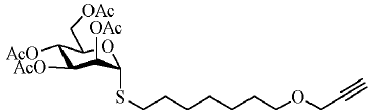

To a solution of **A** (402 mg, 0.995 mmol) and **B** (277 mg, 1.194 mmol) in DMF (41 mL), diethylamine (1.1 mL, 10.8 mmol) was added and the mixture was stirred overnight at room temperature under argon atmosphere. The mixture was evaporated under reduced pressure. The residue was purified by column chromatography (80/20 EP - EtOAc) to give **C** as a colorless oil (321 mg, 63%). The analysis data is consistent with literature. (Dorta et al., ChemBioChem., 17: 936-952, 2016) [9]

### 7-Propargyloxyhelptyl 1-oxo-2,3,4,6-tetra-O-acetyl-α- D-glucopyranoside (E)

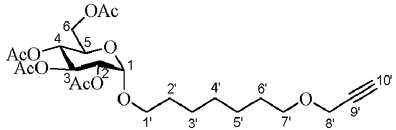

Glucosyl pentaacetate **D** (200 mg, 0.51 mmol), 8-oxaundec-10-yn-1-ol (131 mg, 0.77 mmol) and silver trifluoroacetate (169 mg, 0.77 mmol) were dissolved in dry dichloromethane (3 mL). A solution of SnCl4 1M in dichloromethane (1.53 mL) was added and the mixture was stirred at room temperature for 3 h under argon atmosphere. The solution was diluted with 10 mL of NaHCO₃ (aq) sat. and 10 mL of dichloromethane and the mixture was stirred for 15 min. The mixture was filtrered through a pad of celite, the organic layer was separated and washed with NaHCO₃ (aq) sat. (2×10 ml). The organic layer was dried over MgSO₄ and concentrated under reduced pressure. The residue was purified by column chromatography (8/2 petroleum spirits- ethyl acetate) to give **E** (90 mg, 36%) as a yellowish oil. This procedure allows the formation of the α-anomer.
[α]²⁶_{D}+100 (c 1 ; CH₂Cl₂); ¹H NMR (400 MHz, CDCl₃): δ (ppm): 5.46 (t, *J* = 9.7 Hz, 1 H, H-3), 5.04 (d, *J* = 3.8 Hz, 1 H, H-1), 5.03 (t, 1 H, H-4), 4.83 (dd, *J*_{*2*,*1*} = 3.8 Hz, 1 H, H-2), 4.24 (dd, *J_{6a,6b}* = 12.3 Hz, *J_{6a,5}* = 4.6 Hz, 1 H, H-6a), 4.11 (d, *J* = 2.4 Hz, 2H, H-8'), 4.07 (dd, *J_{6b,5}* = 2.3 Hz, 1 H, H-6b), 3.99 (m, 1 H, H-5), 3.65 (m, 1 H, H-7'a), 3.50 (t, *J* = 6.7 Hz, 2H, H-1'), 3.40 (m, 1 H, H-7'b), 2.40 (t, *J* = 2.4 Hz, 1 H, H-10'), 2.07, 2.04, 2.01, 1.99 (four s, 12H, -CH₃), 1.63-1.54 (m, 4H, H-2', H-4'), 1.40-1.30 (m, 6H, H-3', H-5', H-6'); ¹³C NMR (100 MHz, CDCl₃): δ (ppm): 170.73, 170.28, 170.21, 169.73 (-C=O), 95.79 (C-1), 80.17 (C-9'), 74.18 (C-10'), 71.10 (C-2), 70.42 (C-3), 70.25 (C-1'), 68.83 (C-4, C-7'), 67.30 (C-5), 62.11 (C-6), 58.13 (C-8'), 29.56, 29.31, 29.21 (C-2',C-4', C-6'), 26.17, 26.08 (C-3',C-5'), 20.08, 20.77, 20.73 (-CH₃); HRMS: m/z calcd for C₂₄H₄₀NO₁₁ [M+NH_{4]}⁺calc : 518.2601, found 518.2608.

### General procedure for deacetylation

Acetylated compound was placed in MeOH (10 mL for 1 mmol) with lithium hydroxide (0.5 eq) and the solution was stirred for 1 hour. Water (3 mL for 1 mmol) was added and the mixture stirred for another 30 minutes. Dowex-50 resin was added until pH reached 7. The mixture was filtered through a fritted funnel and concentrated under reduced pressure to yield the deacetylated compound in quantitative yield.

### 7-Propargyloxyheptyl 1-thio-a-D-mannopyranoside (3)

Compound **3** was obtained in quantitative yield (338 mg) by deacetylation of **C** (500 mg, 0.969 mmol) following general procedure for deacetylation.

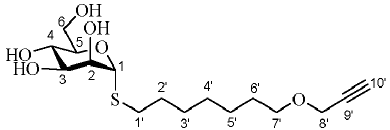

[α]²⁶_{D}+151 (c=1, CH₃OH); ¹H NMR (300 MHz, CDCl₃): 5 (ppm): 5.21 (d, *J_{1,2}* = 1.3 Hz, 1H, H-1), 4.12 (d, *J_{8',10'}* = 2.4 Hz, 2H, H-8'), 3.93-3.86 (m, 2H, H-2, H-5), 3.82 (dd, *J_{6a,6b}* = 11.9 Hz, *J_{6a,5}* = 2.6 Hz, 1 H, H-6a), 3.73 (dd, 1 H, H-6b), 3.67-3.63 (m, 2H, H-3, H-4), 3.52 (t, *J_{7',6'}* = 6.5 Hz, 2H, H-7'), 2.80 (t, *J_{10',8'}* = 2.4 Hz, 1 H, H-10'), 2.74-2.53 (m, 2H, H-1'), 1.70-1.52 (m, 4H, H-2', H-4'), 1.48-1.33 (m, 6H, H-3', H-5', H-6'); ¹³C NMR (75 MHz, CDCl₃): δ (ppm): 86.41 (C-1), 75.53 (C-9'), 74.86, 73.79 (C-2, C-5), 73.19 (C-3), 72.63 (C-10'), 70.94 (C-7'), 68.84 (C-4), 62.74 (C-6), 58.69 (C-8'), 31.81 (C-1'), 30.62, 30.45 (C-2', C-4'), 30.00, 29.78, 27.08 (C-3', C-5', C-6'); HRMS: m/z calcd for C₁₆H₂₇O₆S [M+HCOOH-H]⁻calc : 347.1528, found 347.1531.

### 7-Propargyloxyheptyl 1-oxo-α-D-glucopyranoside (4)

Compound **4** was obtained in quantitative yield (332 mg) by deacetylation of **E** (501 mg, 1 mmol) following the general procedure for deacetylation.

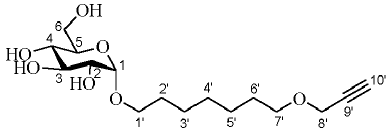

[α]²⁶_{D}+65 (c 1 ; CH₃OH); ¹H NMR (300 MHz, CDCl₃): δ (ppm): 4.77 (d, *J_{1,2}* = 3.8 Hz, 1 H, H-1), 4.12 (d, *J_{8',10'}* = 2.4 Hz, 2H, H-8'), 3.80 (dd, *J_{6a,6b}* = 11.9 Hz, *J_{6a,5}* = 2.5 Hz, 1 H, H-6a), 3.77-3.70 (m, 1H, H-7'a), 3.70-3.60 (m, 2H, H-6b, H3), 3.60-3.54 (m, 1 H, H-5), 3.52 (t, *J_{1'},_{2'} =* 6.5 Hz, 2H, H-1'), 3.48-3.41 (m, 1 H, H-7'b), 3.38 (dd, *J_{2,3}* = 9.8 Hz, *J_{2,1}* = 3.8 Hz, 1H, H-2), 3.29-3.25 (m, 1H, H-4), 2.80 (t, *J_{10',8'}* = 2.4 Hz, 1H, H-10'), 1.70-1.53 (m, 4H, H-2', H-4'), 1.48-1.33 (m, 6H, H-3', H-5', H-6'); ¹³C NMR (75 MHz, CDCl₃): δ (ppm): 100.11 (C-1), 80.87 (C-9'), 75.48 (C-10'), 75.17 (C-3), 73.66 (C-5, C-2), 71.91 (C-4), 71.01 (C-1'), 69.12 (C-7'), 62.74 (C-6), 58.70 (C-8'), 30.55, 30.48, 30.30 (C-2', C-4', C-6'), 27.25, 27.13 (C-3', C-5'); HRMS: m/z calcd for C₁₆H₂₈O₇Na [M+Na]⁺calc : 355.1733, found 355.1724.

### Chemical modifications of cellulose nanofibers (CN)

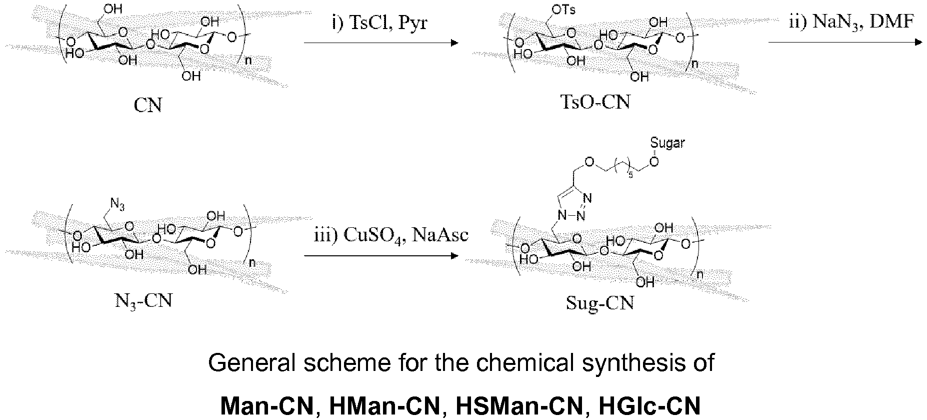

**N₃-CN**. In a first step the primary hydroxyl groups of the glucose units from cellulose nanofibers (CN) are activated with a tosyl group according to method 1 or 2.

Method 1: To a mixture of CN (178 mg, 1 mmol U/Glc) in pyridine (10 mL), *p-*toluenesulfonyl chloride (1.144 g, 6 mmol) was added and the mixture was stirred for 40 hours at room temperature under argon atmosphere. The mixture is filtered through Millipore paper and the CN was washed with ethyl actetate and with DCM to give **TsO-CN** (270 mg, grafting ratio of 50%) as a white solid.

Method 2: To a mixture of CN (178 mg, 1 mmol U/Glc) in pyridine (10 mL), tosyl chloride (1.144 g, 6 mmol) was added and the mixture was stirred for 40 hours at room temperature under argon atmosphere. The mixture is centrifugated for 20 minutes at 40,000 rotations per minute. The supernatant was removed and three other cycles of centrifugation were conducted in DMF. **TsO-CN** was directly engaged in the next step without drying.

**TsO-CN.** Elemental analysis: C: 46.04%, H: 5.08%, N: 0.77%, S: 7.24%; Infra-red analysis (KBr tablet) cm⁻¹: 3377 (*v*(OH)), 2924 (*v*(C-H)), 1542 (*v*(C=C)), 1363 (*vₐₛ*(SO₂)), 1177 (*vₛ*(SO₂))*,* 1059 (*v*(C-O-C)).

Then sodium azide (585 mg, 9 mmol) was added with **TsO-CN** (270 mg, 1 mmol U/Glc) in DMF (10 mL), and the mixture was stirred for 22 hours at 60°C and then 4 hours at 120°C. The mixture was filtered through Millipore paper and the solid washed with water, ethyl actetate and DCM to give **N₃-CN** (197 mg, grafting ratio of 30-40%).

Additional washing was required to remove sodium azide trapped in the CN. **N₃**-**CN** was suspended in water and heated at 80°C under ultrasounds for 20 min. after filtration through Millipore, **N₃-CN** was rinsed with acetone and dichloromethane. **N₃-CN** Elemental analysis: C: 37.55%, H: 4.97%, N: 8.27%, S: 0.00% (SD ∼30-40%); Infra-red analysis (KBr tablet) cm⁻¹: 3377 (*v*(OH)), 2924 (*v*(C-H)), 2108 (*vₐₛ*(N₃)), 1059 (*v*(C-O-C)).

### General procedure for CuAAC

Sodium ascorbate (0.4 eq per N₃ function) and copper sulfate pentahydrate (0.2 eq per N₃ function) was added to a mixture of glycoside (3 eq per N₃ function) and **N₃**-**CN** in dioxane/water 2/1. The mixture was stirred at 60°C for 16 hours. The solvent was removed under reduced pressure, water was added (2 mL) with ethylenediaminetetraacetic acid (EDTA) (2 eq per N₃ function) and the mixture was stirred for 20 minutes to remove the residual copper. The mixture was dialyzed for 24 hours.

**Man-CN** was obtained in quantitative yield (IR analysis) by CuAAC of **N₃-CN** (19 mg, 0.031 mmol of N₃) and propargyl-α-D-mannoside 1 (20 mg, 0.092 mmol) following the general procedure. Elemental analysis: C: 40.23%, H: 5.44%, N: 5.49%, S: 0.00%; Infra-red analysis (KBr tablet method) cm⁻¹: 3385 (*v*(OH)), 2921 (*v*(C-H)), 1059 (*v*(C-O-C)). Substitution degree (SD) 20-30%.

**HMan-CN** was obtained in quantitative yield (FT-IR) by CuAAC of **N₃-CN** (25 mg, 0.040 mmol of N₃) and **2** (40 mg, 0.119 mmol) following the general procedure. Elemental analysis: C: 41.31%, H: 5.88%, N: 4.34%, S: 0.00%; IR analysis (KBr tablet method) cm⁻¹: 3396 (*v*(OH)), 2925 (*v*(C-H)), 1059 (*v*(C-O-C)). SD -30%

**HSMan-CN** was obtained in quantitative yield (IR analysis) by CuAAC of **N₃-CN** (42 mg, 0.067 mmol of N₃) and **3** (70 mg, 0.200 mmol) following the general procedure. Elemental analysis: C: 42.21 %, H: 6.08%, N: 4.34%, S: 2.57%; Infra-red analysis (KBr tablet method) cm⁻¹: 3384 (*v*(OH)), 2924 (*v*(C-H)), 1059 (*v*(C-O-C)). SD ∼30%

**HGlc-CN** was obtained in quantitative yield (FT-IR) by CuAAC of **N₃-CN** (40 mg, 0.063 mmol of N₃) and **4** (63 mg, 0.189 mmol) following the general procedure. Elemental analysis: C: 45.18%, H: 6.33%, N: 4.80%, S: 0.00%; Infra-red analysis (KBr tablet method) cm⁻¹: 3377 (*v*(OH)), 2924 (*v*(C-H)), 1059 (*v*(C-O-C)). SD ∼30%.

### Procedures for the modification of cellulose paper (CP)

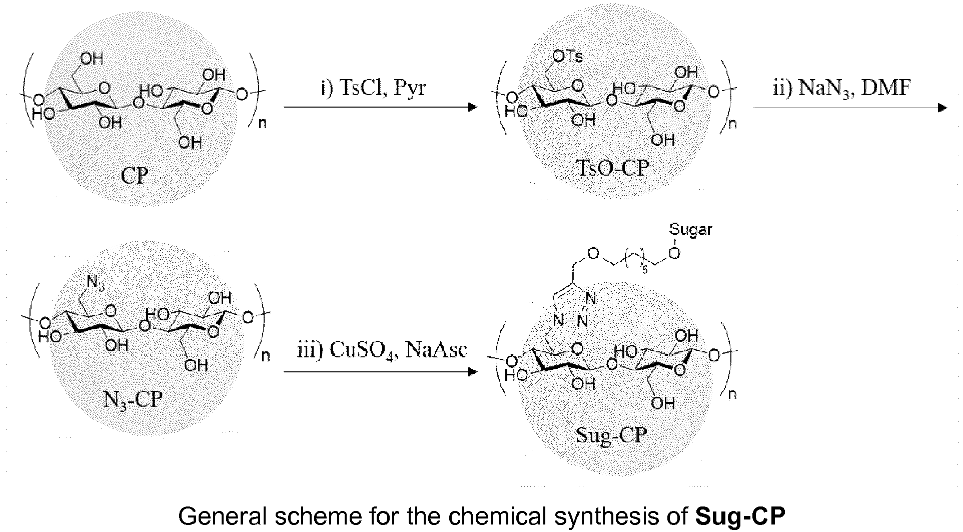

### Procedure for the pre-treatment of CP

The procedures for functionalizing cellulose paper (CP) are very similar to the functionalization of CN. However the chemical modification of CP requires a pre-activation step in order to increase the reactivity of the hydroxyl groups.

To this aim, five pieces of CP (approximately 750 mg) were dispersed in 250 mL of a freshly prepared 10% (w/w) NaOH aqueous solution. This mixture was shaken 24 h on an orbital agitator at room temperature. The cellulose samples were washed 6 times with 50 mL of EtOH until neutrality is achieved, and stored under EtOH.

Once activated, the CP are engaged in tosylation and nitrogenization reactions without being dried (to avoid the re-forming of the hydrogen-bonds network) on an orbital agitator to preserve the CP structure.

### Synthesis of Ts-CP

A piece of CP (145 mg, 0.81 mmol Glc) was immersed in pyridine (10 mL) and treated with p-toluenesulfonyl chloride (464 mg, 2.44 mmol). The mixture was shaken for 20 h at 40°C on an orbital agitator. The paper was sonicated three times with 20 mL of DMF and stored in DMF for the next step. For analytical purposes only, **Ts-CP** was isolated and dried under vacuum. **Ts-CP** is stored in DMF after three successive washings to remove pyridine and unreacted p-toluenesulfonyl chloride. Elemental analysis: C: 46.11%, H: 4.99%, N: 0.13%, S: 6.18%; IR analysis (ATR) cm⁻¹: 3377 (v(OH)), 2924 (v(C-H)), 1542 (v(C=C)), 1363 (*vₐₛ*(SO₂)), 1177 (*vₛ*(SO₂)), 1059 (*v*(C-O-C)).

### Synthesis of N₃-CP

A piece of **Ts-CP** (0.81 mmol Glc) was immersed in DMF (10 mL) and treated with NaN₃ (526 mg, 8.10 mmol). The resulting mixture was shaken for 40 h at 60 °C on an orbital agitator. The **N₃-CP** paper was sonicated with 20 mL of H₂O, acetone, EtOH and DCM and dried under vacuum. Elemental analysis: C: 41.25%, H: 4.86%, N: 6.65%, S: 0.98%; IR analysis (ATR) cm⁻¹: 3377 (*v*(OH)), 2924 (*v*(C-H)), 2108 (*vₐₛ*(N₃)), 1059 (*v*(C-O-C)). SD ∼30-40%.

### Synthesis of HMan-CP

To a mixture of **2** (640 mg, 1.93 mmol) and N₃-CP (0.81 mmol Glc) in dioxane/water 3/1 were added sodium ascorbate (1 eq) and copper sulfate pentahydrate (0.1 eq). The mixture was stirred at 60°C for 24 hours, filtered through a fritted funnel and washed with methanol. The paper was put into water with EDTA (2 eq) and was sonicated. The resulting paper was successively sonicated with 20 mL of H₂O, acetone, EtOH and DCM and dried under vacuum. Elemental analysis: C: 44.63%, H: 6.18%, N: 4.60%, S: 0.95%; IR analysis (ATR) cm⁻¹: 3377 (*v*(OH)), 2924 (*v*(C-H)), 2108 (*vₐₛ*(N₃)), 1059 (*v*(C-O-C)).SD ∼30%.

### EXAMPLE 2 : SPECIFIC DETECTION OF AIEC LF82 USING MANNOSE-FUNCTIONALIZED CELLULOSE

### Bacterial strain and cell line

*E. coli* strain LF82 isolated from a chronic ileal lesion of Crohn's disease patient was used as the AIEC reference strain expressing the mannose-binding FimH adhesin. Bacteria were grown overnight at 37 °C in Luria-Bertani (LB) broth medium.

The human intestinal cell line T84, purchased from American Type Culture Collection (ATCC, CCL-248), was maintained in an atmosphere containing 5% CO₂ at 37°C in the culture medium recommended by ATCC. T84 cells were seeded in 48-well tissue culture plates at a density of 1.5 x 10⁵ cells/well and incubated at 37 °C for 48 h.

### Assessment of CN derivatives for their ability to disrupt attachment of AIEC strain LF82 to T84 cells

**N₃-CN, HMan-CN, HSMan-CN, HGlc-CN, Man-CN** and **HMan** were first incubated at room temperature with AIEC LF82 bacteria for 1h in DMEM/F12 (50/50) medium supplemented with 10% (v/v) heat-inactivated fetal calf serum (FCS) at final concentrations of 100, 10, 1, or 0.1 µM. Mixtures were then added to the T84 monolayers for 3h at a multiplicity of 10 bacteria/cell, at 37°C, in an atmosphere containing 5% CO₂

After infection, cells were washed four time with phosphate-buffered saline (PBS) in order to eliminate non-adherent bacteria CFU (not catched by the CN derivatives). The epithelial cells were then lysed with 1% Triton X-100 (Sigma) in deionized water. Samples were diluted and plated onto LB agar plates to determine the number of colony-forming units. The resulting colony-forming units for the wells treated with the CN derivatives were determined and compared to the value observed with the non-treated wells (NT).

Normalized results expressed as percentage of bacteria associated with the cells for different concentrations of mannose (or N₃ for **N₃-CN**) (n=6 experiments, means±SEMs; *p<0.05, **p<0.01, ***p<0.001) are represented in Figure 2. LF82 infection in the absence of treatment was normalized to 100%.

**N₃-CN** was a poor binder of bacteria because no significant inhibition of adhesion was observed at the high concentration of 100µM. In contrast, **HMan-CN** or **HSMan-CN** showed a strong inhibition capacity with less than 5% of bacteria still adhered to the T84 cells at the highest concentration of 100µM. The dose-dependent inhibition profile was very similar to the one observed with free **HMan** meaning that no loss of inhibitory capacity is observed when the **HMan** or **HSMan** ligands are grafted on the cellulosic framework. In addition, the results of **HSMan-CN** confirmed that thiomannosides were recognized by FimH with a similar affinity than their O-mannoside homologs, even if a slightly weaker antiadhesive effect was observed for the 10 µM concentration.

To further probe that AIEC adherence is fully promoted by the FimH ligands, **HGlc-CN,** the glucose analog of **HMan-CN,** was designed as a negative control. Glucosides are not recognized by FimH and indeed, **HGlc-CN** at 100 µM was totally ineffective in preventing AIEC LF82 binding (Figure 3) further highlighting the exquisite control of FimH in the binding process.

### Assessment of CN derivatives for their ability to capture AIEC LF82 in the complex gut microbiota

**HMan-CN** was studied *in vivo* to evaluate it potency to capture AIEC bacteria in the complex gut microbiota. The non-toxic and biocompatible CN is a particularly suited scaffold for developing a potential treatment against Crohn's disease. The high molecular weight of **HMan-CN** should confine the anti-adhesive in the gut, the locus of infection, and preclude its systemic dissemination, lowering the risk of potential side effects.

The transgenic CEABAC10 mouse model was selected to mimic Crohn's disease susceptibility to be colonized by AIEC bacteria. Indeed CEABAC10 mice express the highly mannosylated CEACAM6 glycoprotein which is overexpressed at the ileal mucosa of patient with Crohn's disease. The mannose residues exhibited by CEACAM6 favor the AIEC attachment to the intestinal mucosa.

Mice were first challenged intragastrically with 5 x 10⁹ AIEC LF82 bacteria. Two hours after infection, **HMan-CN** was orally administered to the mice (n=9 per group) at the dose of 10 mg/kg (on a HM unit basis). A second administration was realized 24 hours later at the same dose. Levels of AIEC bacteria in feces were assessed on days 1 and 2 post-infection for the treated and untreated (NT) mice.

Figure 4 represents the fold decreases in AIEC bacterial colonization in feces on day 1 (D1) and day 2 (D2), relative to the colonization level on day 1 (D1) post-infection of CEABAC10 transgenic mice. The results are expressed in box and whiskers (Min to Max) (**p<0.01, Mann-Whitney test, relative to LF82-infected mice without any treatment (LF82). The results show that the bacterial clearance was more effective in the **HMan-CN** group compared to the LF82 group with 9,63 x 10⁵ vs 3,61 x 10⁶ bacteria/g feces at D2 post-infection, despite a higher bacterial level on day 1 for the **HMan-CN+LF82** group. This shows that **HMan-CN** can bind AIEC in the gut microbiota and could be further evaluate as an anti-adhesive treatment to impact AIEC-induced colitis.

### Detection by paper sensors (Sug-CP) of AIEC from a bacterial culture

The faculty of **HMan-CN** to bind AIEC having been established, the evaluation of potential paper sensors (CP) for AIEC detection was studied.

**HMan-CP** or **N₃-CP** fibers (disc of 6 mm diameter) were pre-incubated in phosphate buffered saline (PBS) at room temperature for 15 minutes with gentle shaking. Fibers were then incubated with bacterial suspensions calibrated at 10⁸ bacteria/mL in PBS for one hour with gentle shaking. AIEC LF82 reference strain, the non-piliated LF82*-ΔfimH* mutant and the laboratory strain *Escherichia coli* K12 C600 were tested for their abilities to bind **HMan-CP** or **N₃-CP** fibers. Fibers were washed 6 times in PBS and homogenized (Ultra Turrax) in 1 mL of PBS. Homogenized fibers were processed to count captured bacteria. Appropriate dilutions of samples were plated onto LB agar. After culturing at 37°C overnight, bacterial counts were recorded. Binding of AIEC to **HMan-CP** or **N₃-CP** fibers was expressed as a percentage of bacteria trapped into fibers.

The results are presented in Figure 5 and show that the AIEC LF82 reference strain is specifically trapped on the cellulose grafted with HMan, whereas the non-piliated LF82-Δ*fimH* mutant and the laboratory strain *Escherichia coli* K12 C600 have very low levels of adhesion to bare or grafted cellulose **(N₃-CP** or **HMan-CP** fibers).

### Detection by paper sensors of AIEC bacteria from feces or intestinal mucosa in mice

Binding ability of bacteria by **HMan-CP** was assessed on feces and intestinal tissues of mice previously infected with AIEC LF82 bacteria. Briefly, human CEACAM6-expressing mice (CEABAC10 transgenic model) were pretreated for 3 days with streptomycin sulfate (2,5 g/L) and DSS (0,5%) in the drinking water then, they were orally challenged with 3.10⁹ bacteria.

### On feces samples :

At 1 dpi, feces were collected and homogenized in PBS at a concentration of 50 mg/ml (Ultra Turrax). Feces suspensions were centrifuged (400 rpm, 5 minutes, room temperature) and the supernatants were collected. **HMan-CP** or **N₃-CP** fibers were incubated with the feces supernatants for one hour at room temperature with gentle shaking. Bound bacteria to the fibers were count as described above. The percentages of LF82 bacteria and of enterobacteria captured by the fibers were determined accordingly to the total number of bacteria per gram of feces (determining by plating onto LB agar containing ampicillin (100 µg/ml) and erythromycin (20 µg/ml) to count LF82 bacteria and on Drigalski agar to count enterobacteria) in the initial sample.

The results are presented in Figure 6 and show that the HM function is necessary for detecting AIEC bacteria (specificity). No enterobacteria from the murine microbiota was trapped (data not shown).

### On colonic mucosa samples :

At 1 dpi, mice were anesthetized with isofluorane and then euthanized by cervical dislocation. Colon was collected, longitudinally opened, washed in PBS and divided into 3 parts (proximal, middle and distal colon). Tissue samples were weighted and homogeneized in PBS at 100 mg/ml. Total LF82 bacteria and enterobacteria were quantified in the supernatants after centrifugation as described previously. In parallel, bacteria trapped into the fibers were quantified (in CFU/cm² of fibers) and the percentages of bacteria binding the **HMan-CP** fibers were determined and compared to percentages of bacteria binding the **N3-CP** fibers.

The results are presented in Figure 7 and show that the HM function is necessary for detecting AIEC bacteria from intestinal tissues.

### List of references

1. Carvalho et al., J. Exp. Med., 206: 2179-2189, 2009
2. Palmela et al., Gut, 67(3): 574-587, 2018
3. Hartmann and Lindhorst, Eur. J. Org. Chem., 2011 (20-21): 3583-3609, 2011
4. Klein et al., J. Med. Chem., 53: 8627-8641, 2010
5. Kleeb et al., J. Med. Chem., 58: 2221-2239, 2015
6. Mydock-McGrane et al., J. Med. Chem., 59: 9390-9408, 2016
7. Jiang et al., J. Med. Chem., 55: 4700-4713, 2012
8. Bouckaert et al., Mol. Microbiol., 55: 441-455, 2004
9. Alvarez Dorta et al., ChemBioChem., 17: 936-952, 2016
10.Sivignon et al., mBio., 6(6): e01298-15, 2015
11.J. Vis. Exp. (88), e5141414, doi:10.3791/5141414, 2014

## Claims

1. Method for detecting *in vitro* pathogenic bacteria expressing a lectin-type adhesin in a subject biological sample, comprising:
a) grafting at least one ligand of a lectin-type adhesin on a cellulose support;
b) contacting the functionalized-cellulose support with the subject biological sample under condition allowing selective adhesion of the pathogenic bacteria expressing the lectin-type adhesin thereon ;
c) detecting the pathogenic bacteria expressing the lectin-type adhesin trapped on the functionalized-cellulose support.

2. Method according to claim 1, wherein the lectin-type adhesin is FimH adhesin.

3. Method according to either one of claim 1 or 2, wherein the ligand of the lectin-type adhesin is a mannose derivative, preferably a heptylmannoside (HMan) or a derivative thereof.

4. Method according to anyone of claims 1 to 3, wherein the subject biological sample to be tested is chosen from the group consisting of bacterial culture, feces, urine, intestinal tissues.

5. Method according to any one of claims 1 to 4, wherein the pathogenic bacteria expressing the lectin-type adhesin are Adherent Invasive *E. coli* (AIECs).

6. Method for identifying a host having a pathology caused by pathogenic bacteria expressing a lectin-type adhesin and mediated by interactions between lectin-type adhesin and host cell surface glycans, comprising using the method for detecting of any one of claims 1 to 5.

7. Method according to claim 6, wherein the pathology is chosen in the group consisting of:
- an inflammatory bowel disease (Crohn's disease, ulcerative colitis, acute diarrhea);
- an urinary tract infection;
- an irritable bowel syndrome;
- colorectal cancer;
- infectious diarrhea.

8. Method according to either one of claims 6 or 7, wherein the lectin-type adhesin is FimH adhesin.

9. A kit for detecting pathogenic bacteria expressing a lectin-type adhesin in a subject biological sample, comprising at least a cellulose support functionalized with a ligand of a lectin-type adhesin, and at least a mean of colorimetric or fluorescence or luminescence detection for detecting trapped pathogenic bacteria expressing the lectin-type adhesin.

10. A kit according to claim 9, wherein the pathogenic bacteria expressing a lectin-type adhesin are Adherent Invasive *E. coli* (AIECs).

11. A kit according to either one of claims 9 or 10, wherein the lectin-type adhesin is FimH adhesin.

12. Cellulose support functionalized with a ligand of a lectin-type adhesin for use in the treatment or prevention of a pathology caused by pathogenic bacteria expressing a lectin-type adhesin and mediated by interactions between lectin-type adhesin and host cell surface glycans.

13. Functionalized-cellulose support for use according to claim 12, wherein the pathology is chosen in the group consisting of:
- an inflammatory bowel disease (Crohn's disease, ulcerative colitis, acute diarrhea);
- an urinary tract infection;
- an irritable bowel syndrome;
- colorectal cancer;
- infectious diarrhea.

14. Functionalized-cellulose support for use according to either one of claim 12 or 13, wherein the lectin-type adhesin is FimH adhesin.
